# EUROPEAN PATENT SPECIFICATION

(11) **EP 3 681 461 B1**
(45) Date of publication and mention of the grant of the patent: **17.03.2021**
(21) Application number: 18756462.0
(22) Date of filing: 28.08.2018
(51) Int. Cl.: A61K 8/27, A61K 8/49, A61Q 5/00, A61Q 5/02, A61Q 5/12, A61K 8/67, A61Q 17/00, A61Q 19/10, A61K 8/19

(54) **AN ANTIMICROBIAL COMPOSITION**
ANTIMIKROBIELLE ZUSAMMENSETZUNG
COMPOSITION ANTIMICROBIENNE

(30) Priority: 11.09.2017 EP 17190375
(43) Date of publication of application: 22.07.2020
(73) Proprietor: Unilever N.V., 3013 AL Rotterdam (NL); Unilever PLC, London, Greater London EC4Y 0DY (GB)
(72) Inventor: DAS, Somnath, Bangalore 560 066 (IN); PRAMANIK, Amitava, Bangalore 560 066 (IN)
(74) Representative: Tansley, Sally Elizabeth
(86) International application number: PCT/EP2018/073082
(87) International publication number: WO 2019/048288

(56) References cited:
- EP-A2- 2 724 707
- WO-A1-2017/097620
- DE-A1-102010 043 076
- US-A1- 2008 145 328
- US-B1- 7 026 308
- DATABASE GNPD [Online] MINTEL; December 2016 (2016-12), "Rapid Lenght Extending Shampoo", XP002776934, Database accession no. 4452545

## Description

### Field of the invention

This invention relates to an antimicrobial composition. The invention more particularly relates to a personal care or cleansing composition e.g. those for care or cleansing of hair or body which provides anti-microbial efficacy. It more particularly relates to a cleansing composition for hair and scalp comprising actives that interact to provide synergistic antimicrobial efficacy for anti-dandruff benefits.

### Background of the invention

The invention relates to an anti-microbial composition useful for cleaning of any surface e.g. an inanimate or a human or animal body part but especially suitable for personal care and cleansing. It further more particularly relates to care or cleansing of the body especially the hair and scalp. Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts. To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g zinc pyrithione (ZPTO), octopirox (piroctone olamine), azole antimicrobials (e.g. climbazole), selenium sulfide and combinations thereof. One of the more popular agent from the above list is ZPTO.

Zinc pyrithione is a potent antifungal agent, however, due to degradation in presence of sebum, its functional efficacy is often compromised. Stabilization of zinc pyrithione is therefore important which can be achieved by suitably combining with certain boosters or by affecting the chemical equilibrium between zinc and the pyrithione moiety.

The present invention relates to a synergistic composition comprising a water-soluble polyvalent metal complex of pyridine amides such as niacinamide, isoniacinamide or picolinamide in combination with a polyvalent metal pyrithione for superior antimicrobial efficacy.

To the knowledge of the present inventors, an antimicrobial composition comprising the above combination of ingredients has not been known or published so far.

A related art is disclosed in WO15198338 which provides for a synergistic antimicrobial composition comprising zinc pyrithione and zinc salt of pyridine carboxylic acid, their tautomeric forms, isomers, polymorphs, salts and solvates and mixtures thereof.

There is still need for newer and more efficacious compositions that synergistically enhance the antimicrobial efficacy of zinc pyrithione.

It is thus an object of the present invention to deliver higher antimicrobial efficacy from metal pyrithiones.

### Summary of the invention

In accordance with a first aspect is disclosed a polyvalent metal salt of pyrithione and a complex of a polyvalent metal with pyridine amide.

Another aspect relates to non-therapeutic use of the composition for delivering antimicrobial benefits.

Other aspects of the invention are discussed in details in the description.

### Detailed description of the invention

According to a first aspect is disclosed an antimicrobial composition comprising a polyvalent metal salt of pyrithione and a complex of a polyvalent metal with pyridine amide.

The polyvalent metal salt of pyrithione is preferably zinc pyrithione (ZPTO) which is shorthand for zinc 1-hydroxy-2-pyridinethione. The polyvalent metal salt of pyrithione is represented by the following general formula:

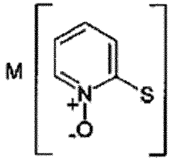

In the case of zinc pyrithione, M is the metal cation zinc.

Zinc pyrithione is preferably present in 0.01 to 10%, more preferably 0.01 to 5.0%, further more preferably from 0.05 to 2.0% based on weight of the composition. ZPTO is a particulate material. While the particle size is not critical to achieve the benefits of the present invention, the particle size of ZPTO is preferably from 0.25 to 8 micrometer, more preferably from 0.5 to 8.0 micrometer, and further more preferably from 1.0 to 7.5 micrometer. ZPTO is commercially available from Kolon Life Science Inc., Sino Lion (USA) Ltd, Lonza, and other suppliers.

The composition of the invention comprises a complex of a polyvalent metal with pyridine amide. The polyvalent metal which is complexed with the pyridine amide is preferably selected from zinc, copper or silver. The most preferred metal for complexation is zinc. The pyridine aminde is preferably selected from one of niacinamide, picolinamide or isonicotinamide.

The structures of the above pyridine amide is given below:

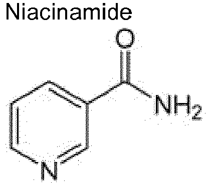

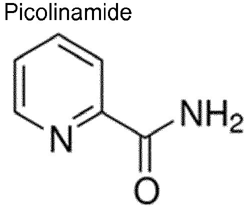

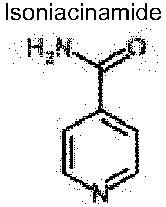

In a pyridine amide (such as nicotinamide) there exists two donor sites through which complexation with metal ion can take place, i) nitrogen atom of the pyridine ring and ii) carbonyl oxygen in the amide functional group. Hence, metal -ligand bonding can occur through any/ both possibilities (if structural conformation permits). It has been reported in literature that bonding through the pyridine nitrogen donor is generally stronger. For a divalent metal ion such as Zn⁺², two molecules for pyridine amide would be required for complexation with one molecule of the metal ion.

The structure of a typical (Zinc) complex is as follows:

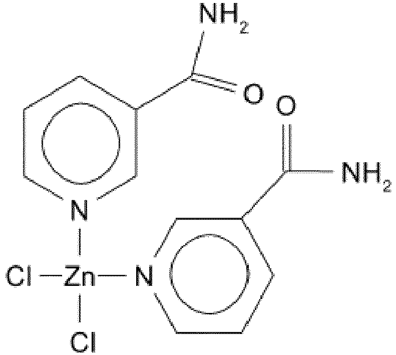

Crystallographic evidences of such complexes are well documented and distinguishable from a physical combination of metal ion and pyridine amide through changes in characteristic IR spectral peak shifts in the complex. References to such complexes have been reported in Journal of molecular structure 605 (2002) 103, Journal of Molecular Structure 794 (2006) 270-276, Computational and Theoretical Chemistry 988 (2012) 27-33.

The preferred pyridine amide for use in the present invention is niacinamide. The composition preferably comprises 0.01 to 10%, more preferably 0.05 to 5%, further more preferably 0.1 to 5% complex of the polyvalent metal with pyridine amide by weight of the composition.

The composition of the invention preferably additionally comprises a cosmetically acceptable carrier. The carrier is preferably chosen such that the composition of the invention can be delivered for use as a personal cleansing composition. As per one aspect the cosmetically acceptable carrier is water or an aqueous solution. According to another preferred aspect, the carrier additionally comprises a surfactant. The cosmetically acceptable vehicle is such that the composition can be prepared as a shampoo, conditioner, body wash, hand wash or face wash product, cream, lotion, gel, powder, ointment, or a soap bar.

According to a further preferred aspect of the present invention, the composition is either a shampoo, a hair conditioner, or a body wash product.

As per an especially preferred aspect of the invention, the composition is a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of the composition is preferably equal to or higher than 4.0, more preferably in the range of 5.0 to 10.0.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives.

Suspending agent, if included, will generally be present in a shampoo composition of the invention at levels of from 0.1 to 10%, preferably from 0.5 to 6%, more preferably from 0.5 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The composition of the invention is preferably aqueous based. It preferably comprises high amounts of water preferably from 70 to 95% by weight of the composition.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

The composition of the invention may be used for skin care e.g. body, hand or face wash. The antimicrobial composition may further comprise a surfactant. The preferred surfactants are nonionic surfactants, such as C₈-C₂₂, preferably C₈-C₁₆ fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Thus, in a highly preferred aspect, the antimicrobial compositions include the surfactant selected from the group of anionic surfactant, fatty acid amide, alkyl sulphate, linear alkyl benzene sulphonate, and combinations thereof.

When the surfactants are present, the antimicrobial composition preferably comprises 1 to 90% surfactant by weight of the composition

When surfactant is used, a particularly preferred surfactant is soap. Soap is a suitable surfactant for personal washing applications of the antimicrobial composition of the invention. The soap is preferably C₈-C₂₄ soap, more preferably C₁₀-C₂₀ soap and most preferably C₁₂-C₁₈ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also be used in other desired proportions.

When present, the soap, of the present is preferably present in an amount of 1 to 90%, preferably from 10 to 85%, more preferably 25 to 75% by weight of the composition.

Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, further more preferably at least 5% by weight of the composition. When water is the carrier, a preferred liquid composition comprises 10 to 99.8% by weight water.

Also disclosed in accordance with this invention is non-therapeutic use of the composition for delivering anti-microbial benefits.

The following non-limiting examples further illustrate preferred embodiments of the invention. All percentages referred to in the examples and throughout this specification are based on total weight unless otherwise indicated.

### Examples

### Examples A-D, 1-3: Antifungal efficacy of compositions as per the invention:

The samples as given in Table - 1 were subjected to their anti fungal efficacy on M. *furfur* as described hereinafter.

### Antifungal activity:

ATCC 14521 strain was revived from glycerol stock on Modified Dixon agar. Plate was at 30°C for 72 hrs. Approximately 72 hrs prior to testing, the plate culture was inoculated onto the surface of another sterile Modified Dixon agar plate and incubated at 30°C for approximately 72 hrs. From second subculture, the optical density of culture is adjusted at 620 nm to give cell strength of 10⁷ cfu/ml. The culture was further diluted by 100 times to achieve 10⁵ cfu/ml in Pityrosporum broth.

10 mg of each sample was thoroughly mixed by vortex and weighed into a sterile 1.5 ml Eppendorf tube. 1000 µL of M. furfur inocula were added to the above tubes and a blank sterile 1.5 ml Eppendorf tube (no particles as control), respectively so that the load in each tube is 10⁵ cells/ml.

All the samples were shaken at 120 RPM in a shaker incubator at 32°C. After 24 hours incubation, 10 µL of the samples were withdrawn from the respective 1.5 ml Eppendorf tubes and diluted with 990 µL of Pityrosporum broth. This represents a 10² dilution. Serial dilution was performed from the above 24 hours inocula and plated 100 µL of dilution onto modified Dixon plate. Plate dilution 10², 10³, 10⁴ and 10⁵5. All the plates were kept for incubation at 32°C for 24 - 72 hrs. Plate count for all the plates were done.

The data on the antifungal activity in terms of log reduction is summarized in Table - 1 below:

**Table - 1:**

| Example | Sample | Contact time, hr | Log reduction |
|---|---|---|---|
| A | ZPTO | 24 | 1.58 |
| B | Zinc isoniacinamide | 24 | 1.98 |
| C | Zinc niacinamide | 24 | 1.96 |
| D | Zinc picolinamide | 24 | 1.92 |
| 1 | Zinc isoniacinamide + ZPTO (weight ratio 1:2) | 24 | 3.20 |
| 2 | Zinc niacinamide + ZPTO (weight ratio 1:2) | 24 | 2.76 |
| 3 | Zinc picolinamide + ZPTO (weight ratio 1:2) | 24 | 2.37 |

The data in Table - 1 indicates that a composition as per the invention (Example 1 -3) provide vastly superior efficacy as compared to those outside the invention (Examples A to D).

### Examples E, F, 4: Anti bacterial efficacy of the composition of the invention:

Compositions as per Table -2 were prepared and they were tested for antibacterial efficacy as per the procedure given below:
40 mg of the powdered sample was dispersed in 100 ml deionised sterile water. The pH was maintained at 10 using NaOH. 8ml of the solution was taken, to it 1 ml cell (1 mL bacterial suspension of Staphylococcus aureus (stock 10⁹ cells/MI) and 1 ml of distilled water was added. The incubation was done at room temperature for 30 and 60 seconds respectively. 1ml of the above reaction mixtures was taken and added to 9 ml of Dey-Engley Neutralizing Broth. This was further incubated for 5 minutes. This represents 10⁻¹ dilution. Serial dilutions were performed for plate count.

**Table 2**

| Example | Sample | Contact time, seconds | Log reduction |
|---|---|---|---|
| E | ZPTO | 60 | 0.5 |
| F | Zinc isoniacinamide | 60 | 0.9 |
| 4 | Zinc isoniacinamide + ZPTO (weight ratio of 1:2) | 60 | 1.6 |

The data in table-2 clearly demonstrates the antibacterial activity of the composition of the invention.

### Examples G, H, 5: Antifungal efficacy of the composition of the invention measured bv MIC (minimum inhibitory concentration):

Compositions as per Table -3 were prepared and they were tested for antifungal efficacy as per the procedure given below:
Disc diffusion technique (agar cup method) was used for measuring the minimum inhibitory concentration of the ZPTO composites. The surface of the agar plates is inoculated with M. furfur ATCC 14521 strain. The plates are swabbed with 100 µl of test inoculum containing approximately 10⁶ cells/ml. The swabbed plates are allowed to dry for 5 mins so to absorb excess of remaining culture fluid. The discs impregnated with different test solution were placed on the inoculated agar surface. Each plate was labelled with different test solution and their respective concentration used. The agar plates are incubated for 72 hrs at 32 °C. Zones of inhibition are measured using calibrated mm ruler.
Culture used: *M.furfur* ATCC 14521
Media used: Modified Dixon agar
Incubation conditions: 72 hrs at 32 C
Diameter of disc used: 10 mm (Himedia make)

**Table 3:**

| **Example** | **Sample** | **test conc.(%)** | **zone of inhibition (mm)** |
|---|---|---|---|
| G | ZPTO | 0.5 | 17 |
| H | Zinc isoniacinamide | 0.5 | 0 |
| 5 | Zinc isoniacinamide + ZPTO (weight ratio of 1:2) | 0.5 | 22 |

The data in table-3 clearly demonstrates the superior antifungal activity of the composition of the invention.

## Claims

1. A composition comprising a polyvalent metal salt of pyrithione and a complex of a polyvalent metal with pyridine amide.

2. A composition as claimed in claim 1 wherein the polyvalent metal salt of pyrithione is zinc pyrithione.

3. A composition as claimed in claim 1 or 2 wherein said complex of the pyridine amide is with polyvalent metal selected from zinc, copper or silver.

4. A composition as claimed in claim 3 wherein said polyvalent metal is zinc.

5. A composition as claimed in any one of the preceding claims wherein said pyridine amide is niacinamide, picolinamide or isonicotinamide.

6. A composition as claimed in claim 5 wherein said pyridine amide is niacinamide.

7. A composition as claimed in any of the preceding claims wherein said composition is a personal cleansing composition.

8. A composition as claimed in claim 7 wherein said personal cleansing composition is a body wash composition, a shampoo or a hair conditioner.

9. A composition as claimed in any one of the preceding claims comprising 0.01 to 10%, preferably 0.01 to 5%, more preferably 0.05 to 2% polyvalent metal salt of pyrithione by weight of the composition.

10. A composition as claimed in any one of the preceding claims comprising 0.01 to 10%, preferably 0.05 to 5%, more preferably 0.1 to 5% complex of the polyvalent metal with pyridine amide by weight of the composition.

11. A composition as claimed in any one of the preceding claims additionally comprising a cosmetically acceptable carrier.

12. Composition as claimed in any one of the preceding claims for use as an antimicrobial composition.

## Patentansprüche

1. Zusammensetzung, umfassend ein mehrwertiges Metallsalz von Pyrithion und einen Komplex eines mehrwertigen Metalls mit Pyridinamid.

2. Zusammensetzung wie in Anspruch 1 beansprucht, wobei das mehrwertige Metallsalz von Pyrithion Zinkpyrithion ist.

3. Zusammensetzung wie in Anspruch 1 oder 2 beansprucht, wobei der Komplex des Pyridinamids mit mehrwertigem Metall ausgewählt aus Zink, Kupfer oder Silber ist.

4. Zusammensetzung wie in Anspruch 3 beansprucht, wobei das mehrwertige Metall Zink ist.

5. Zusammensetzung wie in irgendeinem der vorhergehenden Ansprüche beansprucht, wobei das Pyridinamid Niacinamid, Picolinamid oder Isonicotinamid ist.

6. Zusammensetzung wie in Anspruch 5 beansprucht, wobei das Pyridinamid Niacinamid ist.

7. Zusammensetzung wie in irgendeinem der vorhergehenden Ansprüche beansprucht, wobei die Zusammensetzung eine Körperreinigungszusammensetzung ist.

8. Zusammensetzung wie in Anspruch 7 beansprucht, wobei die Körperreinigungszusammensetzung eine Körperwaschzusammensetzung, ein Shampoo oder eine Haarspülung ist.

9. Zusammensetzung wie in irgendeinem der vorhergehenden Ansprüche beansprucht, umfassend 0,01 bis 10%, bevorzugt 0,01 bis 5%, bevorzugter 0,05 bis 2% mehrwertiges Metallsalz von Pyrithion, bezogen auf das Gewicht der Zusammensetzung.

10. Zusammensetzung wie in irgendeinem der vorhergehenden Ansprüche beansprucht, umfassend 0,01 bis 10%, bevorzugt 0,05 bis 5%, bevorzugter 0,1 bis 5% Komplex des mehrwertigen Metalls mit Pyridinamid, bezogen auf das Gewicht der Zusammensetzung.

11. Zusammensetzung wie in irgendeinem der vorhergehenden Ansprüche beansprucht, zusätzlich umfassend einen kosmetisch verträglichen Träger.

12. Zusammensetzung wie in irgendeinem der vorhergehenden Ansprüche beansprucht zur Verwendung als eine antimikrobielle Zusammensetzung.

## Revendications

1. Composition comprenant un sel de métal polyvalent de pyrithione et un complexe d'un métal polyvalent avec un amide de pyridine.

2. Composition selon la revendication 1, dans laquelle le sel de métal polyvalent de pyrithione est la pyrithione de zinc.

3. Composition selon la revendication 1 ou 2, dans laquelle ledit complexe de l'amide de pyridine est réalisé avec un métal polyvalent choisi parmi le zinc, le cuivre ou l'argent.

4. Composition selon la revendication 3, dans laquelle ledit métal polyvalent est le zinc.

5. Composition selon l'une quelconque des revendications précédentes, dans laquelle ledit amide de pyridine est le niacinamide, picolinamide ou isonicotinamide.

6. Composition selon la revendication 5, dans laquelle ledit amide de pyridine est le niacinamide.

7. Composition selon l'une quelconque des revendications précédentes, dans laquelle ladite composition est une composition nettoyante pour la personne.

8. Composition selon la revendication 7, dans laquelle ladite composition nettoyante pour la personne est une composition pour le lavage du corps, un shampoing ou un après-shampoing.

9. Composition selon l'une quelconque des revendications précédentes comprenant de 0,01 à 10 %, de préférence de 0,01 à 5 %, encore mieux de 0,05 à 2 % de sel de métal polyvalent de pyrithione en masse de la composition.

10. Composition selon l'une quelconque des revendications précédentes comprenant de 0,01 à 10 %, de préférence de 0,05 à 5 %, encore mieux de 0,1 à 5 % de complexe du métal polyvalent avec un amide de pyridine en masse de la composition.

11. Composition selon l'une quelconque des revendications précédentes comprenant de plus un support cosmétiquement acceptable.

12. Composition selon l'une quelconque des revendications précédentes destinée à une utilisation comme une composition antimicrobienne.
